(19)

**Europäisches Patentamt**
**European Patent Office**
**Office européen des brevets**

(11) **EP 4 524 985 A1**

(12) **EUROPEAN PATENT APPLICATION**

(43) Date of publication:
**19.03.2025 Bulletin 2025/12**

(21) Application number: **23197862.8**

(22) Date of filing: **18.09.2023**

(51) International Patent Classification (IPC):
**G16H 20/70** (2018.01)    **G16H 40/20** (2018.01)

(52) Cooperative Patent Classification (CPC):
**G16H 40/20; G16H 20/70;** G16H 30/20;
G16H 50/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **NAUTS, Sanne**
  **Eindhoven (NL)**

• **SAINI, Privender Kaur**
  **Eindhoven (NL)**
• **HEUVELINK, Annerieke**
  **5656AG Eindhoven (NL)**
• **SPELT, Hanne Adriana Alijda**
  **Eindhoven (NL)**
• **BULUT, Murtaza**
  **Eindhoven (NL)**
• **VAN EE, Raymond**
  **Eindhoven (NL)**

(74) Representative: **Philips Intellectual Property &
Standards**
**High Tech Campus 52**
**5656 AG Eindhoven (NL)**

(54) **DEVICE, SYSTEM AND METHOD FOR TRAINING A PATIENT SCHEDULED TO UNDERGO A MEDICAL PROCEDURE**

(57)    The present invention relates to a device (2), system (1) and method for training a patient scheduled to undergo a medical procedure. The device comprises an input (21) configured to obtain procedural information comprising information about the scheduled medical procedure, a processor (22) configured to construct and execute a personalized training program for training the patient, and an output (23) configured to output the control signals to a rendering unit (3).

**FIG.3**

**Description**

FIELD OF THE INVENTION

The present invention relates to device, system and method for training a patient scheduled to undergo a medical procedure. The medical procedure may generally be any medical imaging procedure (e.g. a medical scan), a medical treatment procedure (e.g. a radiotherapy procedure) or any other diagnostic or interventional procedure.

BACKGROUND OF THE INVENTION

Magnetic Resonance Imaging (MRI) and Computerized Tomography (CT) are widely used diagnostic tools in pediatric care that provide doctors with high-quality diagnostic images. However, undergoing a scan (especially an MRI scan) can be stressful, requiring children to lie perfectly still for some time in the range of 10 (CT) to 60 (MRI) minutes in a narrow tunnel of a large device, while it makes odd noises. For healthcare staff, scanning children can provide challenges to their workflow and to the diagnostic quality of the scan. Consequently, anesthesia is used in many hospitals for pediatric scans. Comprehensive patient preparation can reduce the need for anesthesia, but requires a substantial time investment from staff.

For this reason, many hospitals have created child-friendly scan rooms, e.g., by using colored lighting, changing the appearance of the scanner (making it look like a castle or submarine), or allowing children to watch a movie during the scan. Another common approach is to prepare and practice with patients in the imaging facility, for example with an educational video or face-to-face training using a mock scanner or simulated scanning sessions. Some hospitals offer solutions to prepare children at home, such as preparatory videos, brochures and hospital-specific mobile apps. Other imaging facilities combine preparation at home and in the hospital, sometimes in addition to adjustments within the scan rooms. Many of these approaches can improve the scan experience for pediatric patients, while increasing the rate of successful awake scans. It is thus possible to reduce sedation / anesthesia rates in pediatric medical imaging through preparation and guidance.

The Scan Buddy app (released in 2022) was developed to do that: prepare pediatric patients (particularly at the age of 4-8 years) and their caregivers for an MRI scan, in the days or weeks prior to the scan, in the comfort of their home. The iterative design process and a feasibility test of the Scan Buddy app are e.g. described in Saini, P., Koehn, C., Heuvelink, A., Tasar, O., van Vorstenbosch-Lynn, E., Nauts, S., & Trout, A. T. (2022, June). Scan Buddy: A Gamified App to Prepare Children for an MRI Scan. In Human-Computer Interaction. Theoretical Approaches and Design Methods: Thematic Area, HCI 2022, Held as Part of the 24th HCI International Conference, HCII 2022, Virtual Event, June 26-July 1, 2022, Proceedings, Part I (pp. 594-612). Cham: Springer International Publishing. The Scan Buddy app provides a learning framework based on informing, familiarizing and training children for MRI and represents a gamified app with different learning goals.

SUMMARY OF THE INVENTION

It is an object of the present invention to further improve preparation of a patient scheduled to undergo a medical procedure.

In a first aspect of the present invention a device for training a patient scheduled to undergo a medical procedure is presented, the device comprising:

an input configured to obtain procedural information comprising information about the scheduled medical procedure;
a processor configured to construct and execute a personalized training program for training the patient, including

- constructing an initial training program based on the obtained procedural information;
- executing the initial training program;
- obtaining performance information indicating the performance of the patient in the training;
- updating the training program based on the obtained performance information; and
- generating control signals for controlling a rendering unit to render visual and/or audible information according to execution of the training program,

wherein the obtained procedural information is used for constructing the initial training program and/or for updating the training program; and
an output configured to output the control signals to the rendering unit.

In a further aspect of the present invention a system for training a patient scheduled to undergo a medical procedure is presented, the system comprising:

- a device for training a patient scheduled to undergo a medical procedure as disclosed herein; and
- a rendering unit configured to render visual and/or audible information according to control signals received from the device.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to personalize the device, system and method to individual patients rather than providing the same learning experience to all patients. The proposed solution is flexible and adaptive to individual patient needs and allows optimal preparation of patients, e.g. at home, for a medical procedure such as an MRI or CT scan, or another diagnostic procedure (such as ultrasound, X-ray, fluoroscopy, PET-CT, etc.) or interventional procedure (such as radiotherapy, proton therapy, IGT-procedures, dentistry, vaccination, blood collection, etc.) or any other medical procedure that may be perceived as scary by a patient. Generally, the present invention is applicable for any medical procedure where personal coaching (or, in other words, patient preparation) improves the outcome of the medical procedure. Preferred areas of application are preparation for any medical procedure that a child is scheduled to undergo.

The training program can be adapted to the specific preparation needs of the particular patient for the scheduled medical procedure in on or more aspects including e.g. content, learning goals, level of difficulty, etc. Thus, personalized, tailored experiences are presented to the patient based on the specific medical procedure the patient is scheduled for.

In this context, the expressions "scheduled for" and "scheduled to undergo" shall be understood broadly. Generally, someone (e.g. a caregiver, nurse, physician, etc.) tells the patient or plans which medical procedure to undergo so that the patient is scheduled to undergo the medical procedure, most often some longer time in advance. But even if a patient is not scheduled for a medical procedure long in advance but is coming to an emergency department and has to quickly undergo a medical procedure, one may still want to prepare the patient for the medical procedure in an adaptive and personalized way if possible. For instance, a child coming into the emergency department may be prepared while waiting for the medical equipment to become available, or a child coming out of an imaging scan with e.g., a suspected tumor and being send from one diagnostic imaging modality to another medical procedure, may still be prepared for the next medical procedure. Thus, the expression "scheduled" does not mean that the medical procedure is scheduled a certain minimum time in advance, but generally in any case where it is decided or asked that the patient shall undergo a medical procedure, it shall be understood that the patient is scheduled for the medical procedure, irrespective of the remaining time up until the medical procedure is carried out.

According to an embodiment, the processor is configured to construct the initial training program by selecting and ordering one or more training modules and one or more content components based on the obtained procedural information, in particular by selecting and/or adapting and/or ordering one or more of learning goals, difficulty level, prioritization and content of the one or more training modules and/or the one or more content components based on the obtained procedural information. Thus, already the initial training program is adapted to the patient and the planned medical procedure, rather than providing the same general initial training program. This helps improving the patient's preparation.

According to another embodiment, the processor is configured to update the training program by adapting one or more of difficulty level, prioritization, content, level of detail, style of presentation, semantics, words, sounds and environment of the one or more training modules and/or the one or more content components based on the obtained performance information, in particular based whether or not and/or to which extent one or more learning goals have been achieved. Updating the training program further improves patient preparation since reactions/performance of the patient during execution of the training program (which includes execution of the initial training program and the updated training program) will be considered for further adapting the training program in or more aspects to the particular patient.

The steps of selecting, ordering and/or adapting various parameters of the one or more training modules and one or more content components may be done based on a look-up table. For instance, the style and/or detail-level may be taken from the look-up table. Alternatively, the training module may be re-done as a kind of trial and error and it may be checked if there is a better score. Alternatively, artificial intelligence algorithms may be used for this purpose.

The input may be configured to obtain, as procedural information, information regarding one or more of

- the type of medical procedure,

- the body part of the subject to undergo the medical procedure,
- duration and/or time of the day of the medical procedure,
- details of the medical procedure,
- requirements of the patient during the medical procedure, in particular regarding breath holding, remaining akinetic, and posture,
- travelling time and/or waiting time of the patient before undergoing the medical procedure, and
- the requirement of taking medication and/or administering contrast agent.

Details of the medical procedure may e.g. include information about the specific type of scan or treatment. Such detail may e.g. be found on an exam card (e.g. an exam card for MRI containing information about the MRI sequences to be run).

The input may further be configured to further obtain patient information comprising information regarding the patient and/or environment information comprising information regarding the environment in which the patient will undergo the scheduled medical procedure. The processor may then be configured to additionally use the obtained patient information and/or the obtained environment information for constructing and/or updating the initial training program. The additional use of patient information and/or environment information further improves the preparation of the patient since it even more personalizes the training.

Patient information may include information regarding one or more of age, size, weight, gender, health state, medication, medical history, preferences, circadian rhythm, chronotype, pain, sensitivity, problems during a prior medical procedure, frequency and/or intensity of problems during a prior medical procedure, user preferences (e.g., information about a child's favorite color), prior experience with medical procedures (e.g. whether this is the patient's first medical procedure of this type or in general), personality, coping style, mood, frequent emotional states, and/or communication preference (e.g. preference for a direct style of communication or not).

Environment information may include information regarding the room in which the patient will undergo the medical procedure including one or more further devices present in the room, colors in the room, persons in the room, layout of the room, clothes worn by the persons and/or the patient during the medical procedure, and other details of the room. This information serves to make the patient familiar with the environment, for instance with how the devices in the room look like, what they are, where they are located, which noise and/or heat they produce, etc.

In another embodiment the input is configured to obtain the procedural information and/or the patient information and/or the environment information from one or more of a user interface configured to receive user input, an exam card, a medical record, and a database. Thus, generally any source of information may be used to obtain (i.e. retrieve or receive) the desired information for personalizing the training program.

The performance information that is obtained during execution of the training program may include information regarding one or more of

- breath holding,
- remaining akinetic,
- being in a predetermined or the same posture,
- required time for solving a task,
- achieved difficulty level,
- coping with emotions including one or more of stress, fear, and anxiety,
- achievements of learning goals,
- physiological measurements including one or more of heart rate, respiration rate, anxiety level, and skin conductance,
- psychological measurements,
- speech,
- sound,
- image and/or video data, and
- number and/or type of errors on a task (e.g. how many and what kind of mistakes does the patient make).

Coping with emotions may e.g. be validated by questionnaires or methods to measure these emotions that can be employed for monitoring during the personalized training. Psychological measurements may be estimated from vital signs of the patient or questionnaires or direct input from the patient. Speech, sound, image data and/or video data may be collected from the patient and used to evaluate physiological and psychological states.

There are multiple options to use the performance of the patient in the training. Preferably, the processor may be configured to perform one or more of the following operations:

- determine a patient score indicating the preparedness level of the patient for the scheduled medical procedure;
- determine if the patient needs extra support and which type and level of support;
- scheduling a visit of a doctor or caregiver;

- re-schedule the scheduled medical procedure;
- provide one or more rewards to the patient when one or more learning goals are met;
- suggest one or more further activities of the patient or a caregiver;
- generate and execute interaction, in particular questions and/or suggestions, with the patient and/or a caregiver;
- select content to be presented to the patient;
- collect information to be provided to a caregiver or other person;
- generate a code, identifier or link to information about the performance of the patient and/or regarding parameters that may be used during the scheduled medical procedure.

If and which of these options is used may be decided based on the available information (procedural information, patient information, environment information, and/or performance information) with the intention to further personalize the training.

In another embodiment, the processor may be configured to additionally use, for constructing and/or updating the initial training program, one or more of:

- time of the day,
- time up to the scheduled medical procedure,
- required quality of an imaging procedure as scheduled medical procedure,
- likelihood that a caregiver or other person accompanies the patient at the scheduled medical procedure, and
- a predetermined protocol.

Such factors may have an influence on the patient's performance and the preferred way of training the patient. Taking such factors into account further contributes to making the training more effective.

The proposed system comprises, in addition to the disclosed device, a rendering unit configured to render visual and/or audible information according to control signals received from the device. The rendering unit may comprise a display configured to display visual information to the patient and/or a loudspeaker to output audible information to the patient. The system may be implemented in a computer or a game console or a handheld device, in particular a smartphone, laptop or tablet. Generally, any programmable device may be used for implementing or including the proposed system and device and for executing the proposed method.

The system may include one or more of the following elements:

- a user interface configured to receive user input of the procedural information and/or patient information;
- a procedural information database containing procedural information comprising information about medical procedures including the scheduled medical procedure;
- a patient information database containing patient information comprising information regarding the patient;
- a content components database containing a plurality of content components for constructing a personalized training program, in particular comprising one or more of game components, video components, images, spoken and/or written text components, sound components, movie components for different medical procedures including the scheduled medical procedure, game asset modules, and educational modules;
- a training module database containing a plurality of training modules for constructing a training program; and
- one or more measurement elements configured to measure the patient's performance in the training.

There are generally different options to implement the steps of constructing the initial training program and updating the constructed initial training program. According to a first option 1, a first customization of the training program (the construction of the initial training program) is done based on the procedural information and the second customization (the updating of the training program) is based on individual characteristics (emotional intelligence, learning performance, personality, context, etc.). According to a second option, the first customization is done based on the individual characteristics (e.g. patient information), and the second customization is done based on the procedural information. According to a third option, it may be started with equal weight (hybrid option) for each of these two aspects of procedural information and individual characteristics, and then later (for the updating) more weight may be given to one of them.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention;
Fig. 2 shows a schematic diagram of an embodiment of a device according to the present invention;

Fig. 3 shows a schematic diagram of an embodiment of a method according to the present invention;
Fig. 4 shows a schematic diagram of an implementation of a system according to the present invention in the form of a mobile phone; and
Fig. 5 shows a schematic diagram of another embodiment of a method according to the present invention.

DETAILED DESCRIPTION OF EMBODIMENTS

In the following the present invention is largely illustrated using an example for the case when a child between 4 and 8 years is scheduled to undergo a scan such as an MRI examination. Of course, the present invention may be adapted for other medical procedures and patients, such as children of other age groups or adults as well. In some cases alternatives are mentioned, but if this is not the case, then the example is not limiting the scope of the claims to exclude any other age groups or medical procedures.

In other word, any reference to a child shall be understood as generally meaning a patient. Any reference to a scan or exam shall be understood as generally referring to a medical procedure. Any reference to an app shall be understood as generally referring to a training program.

Having an MRI scan can make children anxious and prevent them from keeping still for a scan, and in general be less than cooperative. This has a negative impact on the diagnostic quality of the images and may increase the need for costly repeat scans. For this reason, many young children require sedation or general anesthesia (GA) to undergo a scan. However, using sedation/GA is costly: a scan with GA is approximately 9 times more expensive than an awake scan, in particular due to fewer required resources (no hospital bed, no anesthetic or personnel needed to provide GA, no additional time needed before a scan, and shorter waiting lists). There are studies that suggest that sedation/GA can have potentially adverse health effects. Preparing a patient for an awake scan can thus avoid unnecessary exposure to general anesthesia.

Digital tools such as smartphone applications (e.g., the Scan Buddy app) can provide hospitals with a scalable way to prepare patients. Digital tools can be used in combination with live, face-to-face preparation to augment it, or (in cases in which face-to-face training is unavailable), digital tools can be used instead of face-to-face preparation. However, existing digital applications are generic and one-size-fits-all, they are not tailored to an individual patient's specific imaging procedure and needs. As such, not all children receive the optimal preparation for their specific medical procedure.

An insight underlying the present invention is that patients that are scheduled to undergo a medical procedure will understand what is going to happen and what is required from them better and/or more efficiently if the information is supplied in a more interactive and engaging manner, which requires the patient to perform actions that cause the patient to immediately experience the consequences. A further insight is that the patient will be better prepared and more familiar with the scheduled medical procedure if the information the patient receives relates directly to the scheduled procedure (improving so-called transfer of learning). A still further insight is that a personalized, tailored experience is most efficient to prepare the patient to the scheduled procedure.

Compared to a generic one-size-fits-all app, an app (or, more generally, a training device and method) that is tailored and personalized will do a better job of preparing patients for their medical procedure. It can prepare patients for exactly those things that are relevant to them. It can provide a personalized learning experience that prepares patients for exactly those things that they need to know/master to the extent that they need to master them.

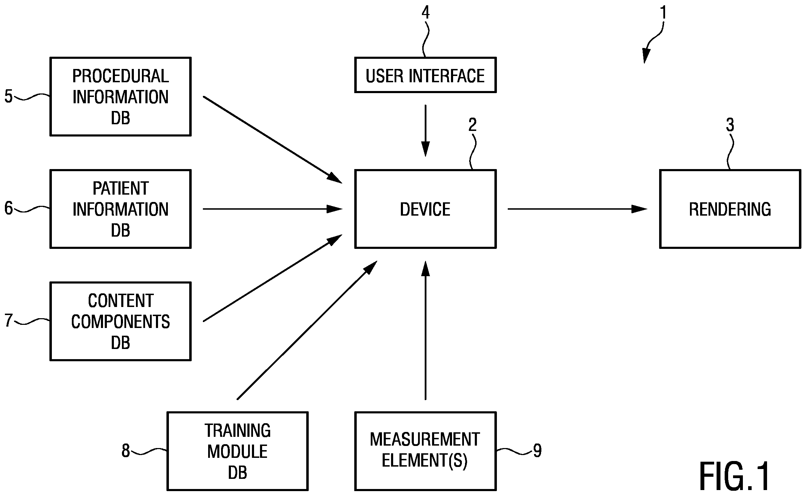

Fig. 1 shows schematic diagram of an embodiment of a system 1 for training a patient scheduled to undergo a medical procedure according to the present invention. The system comprises a device 2 for training a patient scheduled to undergo a medical procedure as will be explained in more detail below and a rendering unit 3 configured to render visual and/or audible information according to control signals received from the device 2.

The system 1 may be implemented in a computer or a game console or a handheld device, in particular a smartphone, laptop or tablet.

The device 2 may generally be implemented by respective units or circuitry, e.g. a processor, processing circuitry, a computer, dedicated hardware, etc., that carries out the functions of the device. Alternatively, a common unit or circuitry, e.g. a common processor or computer, may implement the various functions of the device, or separate units or elements may be used that together represent the circuitry. In an exemplary implementation, a programmed processor may represent the device 2, which may execute a computer program that may be stored in a memory that is accessed by the processor.

The rendering unit 3 may generally be any means that outputs visual and/or audible information, e.g. in text form, as image or diagram, as sound or spoken words, etc. For instance, the rendering unit 3 may comprise (or be implemented as) a display configured to display visual information to the patient and/or a loudspeaker to output audible information to the patient. Exemplary implementation may use a touchscreen, a computer monitor, the screen of a smartphone or tablet, etc. as rendering unit 3.

The system 1 may further comprise a user interface 4 that is configured to receive user input, e.g. of procedural information and/or patient information. The user interface 4 may e.g. comprise a keyboard, touchscreen, microphone or any other entity that allows a user to enter information.

The system 1 may further comprise a procedural information database (DB) 5 containing procedural information comprising information about medical procedures including the scheduled medical procedure. The procedural information database 5 may be stored on a server, central storage of a hospital or medical service, in the cloud or at another location that is accessible by the device 2. The procedural information may e.g. be information about scan modality (such as MRI or CT), information about the body part to be scanned, scan sequences (duration; breath hold y/n; etc.), information whether or not contrast agent it required, planned orientation of the patient, etc.

The system 1 may further comprise a patient information database 6 containing patient information comprising information regarding the patient. The patient information database 6 may be stored on a server, central storage of a hospital or medical service, in the cloud or at another location that is accessible by the device 2.

The system 1 may further comprise a content components database 7 containing a plurality of content components for constructing a personalized training program, in particular comprising one or more of game components, video components, images, spoken and/or written text components, sound components, movie components for different medical procedures including the scheduled medical procedure, game asset modules, and educational modules. The content components database 7 may be stored on a server, central storage of a content provider, in the cloud or at another location that is accessible by the device 2. The content components database may be able to generate further content or provide the content to another entity for generating further content. For instance, the existing content may be used to generate new content, e.g. by applying predetermined content generation rules and/or methods, which may be part of the content components database as well.

The system 1 may further comprise a training module database 8 containing a plurality of training modules for constructing a training program. The training module database 8 may be stored on a server, central storage of a training module provider, in the cloud or at another location that is accessible by the device 2.

The system 1 may further comprise one or more measurement elements 9 configured to measure the patient's performance in the training. These measurement elements 9 may e.g. comprise a processor that measures reactions of the patient performing the training (e.g. reaction time, kind of reaction, etc.), a video camera that monitors the patient performing the training, a score calculation means, etc.

Two or more of the databases 5 to 8 may also be combined into common databases and/or stored at the same location, such as the cloud or archive or hospital's network.

The content components database 7 may be a database with a large variety of training module (also called app module) content components, preferably properly tagged with their content, to enable the construction of a personalized app experience. Examples of content components that may be part of the database are:

- various games, videos, images and spoken/written text that match specific learning goals (e.g., games that serve to teach children about "being familiarized with the sounds of the scanner", "understanding the importance of lying still", and/or "understanding that metal is not allowed into the MRI room");
- various scan sounds to personalize the digital intervention with the right (e.g., MRI or CT) sounds, and optionally the expected order of sounds;
- movies or movie clips for the various scan modalities, body parts, procedure steps to offer a movie embedding only correct and relevant information for the scheduled procedure;
- digital game assets such as models of MRI or CT scanners and scan accessories to enable displaying and interacting with these elements in a game catered to the type of modality and body part to be scanned; and
- educational materials to cater for scan modality and body part (e.g., education items to present to children during game play, information for parents/caregivers; frequently asked questions, etc.).

The training module database 8 may be database of generic training modules (also called 'app modules') in the form of interactive games with game play elements whose difficulty level or threshold for success can be adapted (e.g., the time or accuracy with which an object or body part has to be held still) and a method to tailor this difficulty level given the required level and the patient learning curve deduced from the game play data. To give an example, a lying still game in which children need to keep their phone still can have a low or high threshold for movement and it can differ in the time a patient needs to hold the phone still, etc.

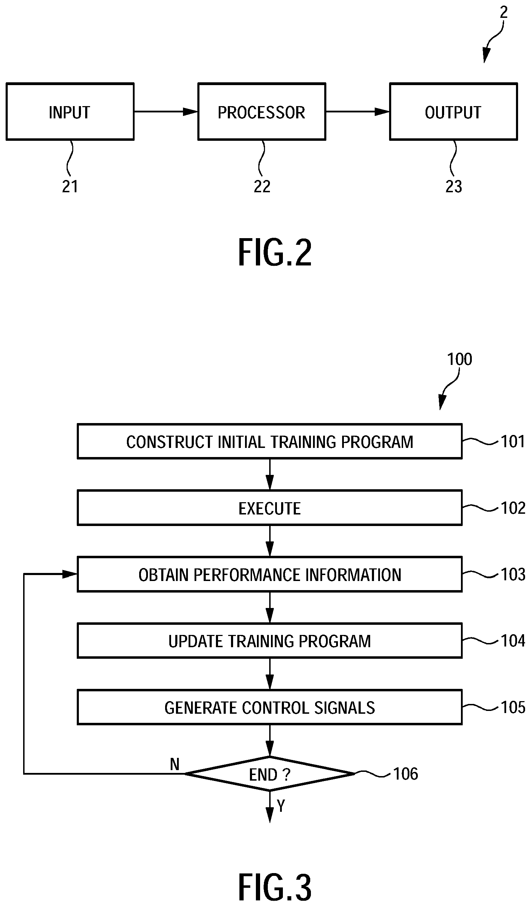

Fig. 2 shows a schematic diagram of a device 2 for training a patient scheduled to undergo a medical procedure. The device 2 comprises an input 21 configured to obtain procedural information comprising information about the scheduled medical procedure, a processor 22 configured to construct and execute a personalized training program for training the patient and an output 23 configured to output the control signals to the rendering unit 3.

The input 21 may be directly coupled or connected to the user interface 4 or to the required one or more of the databases 5 to 8 or to one or more measurement elements 9 in order to obtain (i.e. retrieve or receive) the required information. The information may also be stored in a storage, buffer, network, or bus, etc. The input may thus e.g. be a (wired or wireless) communication interface or data interface, such as a Bluetooth interface, Wi-Fi interface, LAN interface, HDMI interface, direct cable connection, or any other suitable interface allowing information transfer to the device 2.

The processor 22 may be any kind of means configured to process the information and determine control signals there from. It may be implemented in software and/or hardware, e.g. as a programmed processor or computer or app on a user device such as a smartphone, smartwatch, tablet, laptop, PC, workstation, etc.

The output 23 may generally be any interface that provides the determined control signals, e.g. transmits them to another device or provides it for retrieval by another device (e.g. a smartphone, computer, tablet, etc.). It may thus generally be any (wired or wireless) communication or data interface.

Fig. 3 shows a schematic diagram of an embodiment of a method 100 for training a patient scheduled to undergo a medical procedure according to the present invention. The steps of the method 100 may be carried out by the device 2, wherein the main steps of the method 100 are carried out by the processor 22. The method 10 may e.g. be implemented as computer program running on a computer or processor.

In a first step 101 an initial training program (for instance in the form of an 'app' or 'application') is constructed, for instance based on the procedural information that is obtained by the input 21 or, in addition or alternatively, patient information about the patient. In a second step 102 the initial training program is executed. In a third step 103 performance information indicating the performance of the patient in the training is obtained. In a fourth step 104 the training program is updated based on the obtained performance information and, optionally, based on the procedural information. The updated training program is then executed. In a fifth step 105 control signals are generated for controlling the rendering unit 3 to render visual and/or audible information according to execution of the training program. Steps 103 to 105 may be continuously / iteratively executed until an end criterion is reached, which may be checked in step 106. An end criterion may e.g. be that the user stops executing the training program or that a desired goal of the training program has been reached or that a time-out is reached.

Generally, the procedural information can be generated through manual input (e.g. from staff), or it can be taken automatically from medical records (e.g., EMR systems), information systems, or other information sources. These information sources can be combined. The procedural information can be automatically pushed to the device, or it can be encoded in a link to an app running on the device 2 (e.g., it can be part of the information provided to patients/their caregivers). For example, patients may receive a (digital) invitation to the scan that includes a QR code containing information about the scheduled procedure.

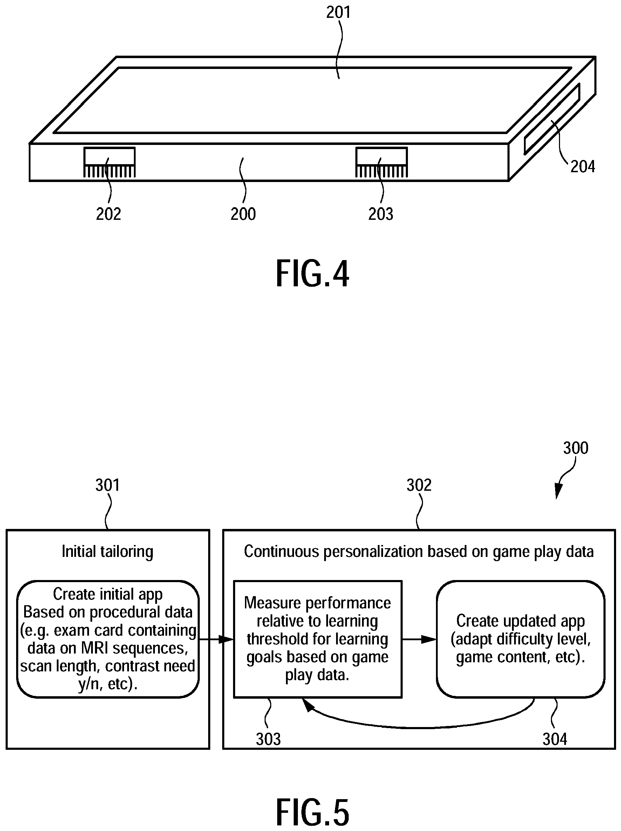

Fig. 4 shows an exemplary implementation of a system 1 according to the present invention as a mobile phone (in particular as a smartphone) 200 having a display screen 201, an integrated processor 202, a motion sensor 203 and a loudspeaker 204.

The mobile phone 200 may be of any size, shape, model, etc., as long as it is suitable to handle the requirements of being used as a training system as disclosed herein.

The display screen 201 may be of any size or type (e.g. LCD, LED and the like). Screens with a large surface area and the capability of displaying strong colors or small and precise details are preferential. Preferably, the display 201 is integrated in the mobile device 200, but the visual content may also be duplicated or transmitted to an external display screen connected by wire or wirelessly to the rest of the handheld device, for instance a remote display screen, goggles or glasses, or any virtual reality device (e.g. virtual reality glasses). An integrated screen 201 is the most practical and compact solution, but an external screen, which by definition in the context of the presently claimed invention would still be part of the handheld device, even though that external screen may not be meant to be handheld or portable itself. Such an external screen may be easier to use in case the patient is not able to watch an integrated screen, e.g. due to the patient's position or other reason that prevents the patient from holding the handheld device and watching the integrating screen at the same time.

The processor 202 shall be capable of receiving, processing and analyzing incoming information and outputting visual and/or auditory information, in real time. The processor may also be in contact with a remote processor, for instance through the internet or through a wired or wireless, such as Bluetooth or Wi-Fi, connection with a local external processor, e.g. a workstation that may support the processor in processing power and/or may provide additional information, such as patient data and/or may store data of training sessions to be processed and/or reviewed automatically, by a physician for use in future sessions or to evaluate if the patient is sufficiently trained to undergo the medical procedure.

The motion sensor 203 shall be sensitive enough to detect relatively small and large motions caused by the user holding the handheld device 200. Most handheld devices are equipped with a motion sensor. These are usually relatively simple accelerometers, but more advanced accelerometers or other integrated motion sensors that would be suitable are possible as well.

It is preferable that the handheld device 200 is capable of providing auditory information, such as sound effects, music, coaching or other informative spoken information and the like to make the experience more immersive and effective. For this purpose, the mobile phone 201 is equipped with an integrated loudspeaker 204. Alternatively or simultaneously a headphone connection may be available to allow a headphone worn by the patient to be connected. The visual information, optionally augmented with auditory information, is preferably in the form of an (interactive) movie, a game or a manipulatable image. The visual information may be relatively simple, but also may be complex and rich in content.

The term 'handheld device' shall be understood generally as covering any suitable device that is meant to be held by a user when in use, irrespective of whether the patient is actually holding the device or not. The handheld device is preferably a mobile phone as this is a device most people own or have direct access too. In other embodiments the handheld device may also be a tablet computer or a gaming device (e.g. a controller for a gaming computer or a mobile gaming computer).

The handheld device shall be able to detect information regarding the performance of the user during the training, e.g. to detect motion, reaction times, user operations, etc. Further, it shall be able to provide visual and/or auditory information to the user. Mobile phones, and particularly smartphones, which are standardly equipped with a display screen and a motion sensor are easily available, as are many types of tablet computers and gaming devices (although in those cases the display may be an externally connected display).

As most people own or have access to a suitable handheld device, they can be supplied with the software to run the training on their handheld device. If not, then they may be given a mobile device temporarily to use the training. An advantage is that the patient can do the training with the training device at a place and time which is convenient for them, for instance at home.

In the context of the present invention the term 'holding' should not be interpreted as that the patient is holding the handheld device only just by holding it using one or both of his or her hands, but also encompasses any other way in which the patient can control the balance of the handheld device. For instance, some patients may not be able to hold a device in one or both of their hands in a steady manner or the patient may not be able to hold the handheld device at all, but they might be able to lie still with their head, torso or other body part(s) that need to be still during the procedure. In those cases the patient may, for instance, 'hold' the handheld device by balancing it on other body parts, preferably those most relevant for the upcoming procedure, such as the torso (i.e. belly or back when lying down), knee, leg, arm, feet, head et cetera, as long as the patient is able to influence mobility of the handheld device and receive feedback thereof

Fig. 5 shows a flowchart of another embodiment of the method 300 according to the present invention, in particular for creating a personalized app (or computer program) for training a patient. It comprises several components to personalize the digital intervention via the app in order to optimally prepare the patient for their upcoming medical procedure, e.g. an upcoming scan. Hereby, tailoring and personalization can be done using two steps.

In a first step 301, an initial app is created that is tailored to a patient and his/her procedure. This may be done with tailored app modules (like videos and games) to include only those learning goals relevant to the patient's specific procedure. The app modules may be tailored in terms of difficulty level and prioritization (e.g., order) of content to help patients reach learning goals at the level/threshold required for a specific procedure and in terms of content (e.g., specific MRI/CT sounds, components of the device using for the procedure, like coils of the MRI scanner, X-ray source of the CT scanner, etc., that are made part of the videos/games) so that they represent the procedure the patient will undergo.

In a second step 302 the app is dynamically personalized based on information obtained while the patient is executing the app, also called performance information (or play data). This includes measuring the performance in sub-step 303 and updating the app in sub-step 304. The personalization may be made with respect to one or more aspects: personalization of the difficulty level of (sub-)games, order of those (sub-)games, incentives provided to play certain (sub-)games, and/or exact educational content provided within each (sub-)game. For instance, in an augmented reality (AR) module, only educational content is shown for those learning goals for which a threshold of a certain performance set for the respective learning goal has not yet been reached.

In another embodiment, a personalized app can be created in two phases. First, the initial app is tailored based on procedure-specific requirements. Next, the app is continuously, and possibly automatically, updated and adapted based on the patient's performance during app use, relative to their individualized performance threshold, for all learning goals relevant to that particular patient. In other words, a feedback loop automatically takes into account the result of a success measure and adapts the presented contents.

In another embodiment, tailored instantiations of generic app modules may be generated by matching tags of the content of the content components database 7 with tagged content of various app components with the information on the scheduled procedure.

According to the present invention a personalization of the training program is made, which may include customization of content and/or design. It can be distinguished between two similar, but distinct concepts: tailoring and personalization. Tailoring refers to segmentation in groups of users (e.g., age, gender, scan type). Personalization refers to segmentation on individual level. This often involves some kind of continuous input data or feedback loop. Generally, user characteristics can be fixed, uncertain or variable allowing for fixed or adaptive.

Embodiments of the present invention include two essential aspects: fixed tailoring based on procedure-specific information (such as scan type or exam card); and a feedback loop, possibly automatic, that considers variable parameters and allows for adaptive content based on the patient's learning curve in the training program. Tailoring is used to create the initial version of the training program that will be presented to a patient. This training program can then be continuously adapted based on play data derived from the training program. Both aspects (fixed tailoring and dynamic personalization) will be described in more detail below.

According to a first aspect, fixed tailoring based on procedure-specific parameters ('procedural information') is applied, which is discussed in the following. Different medical procedures, e.g. medical exams, have different characteristics: some exams are short, while others take a long time; the sounds a scanner makes differ between exams; some exams (e.g., MR) require that all metal is removed before entering the exam room, while other exams do not require this; etc. An app will be more effective if it is tailored to the patient's specific exam. For example, receiving contrast is a stressful, but often necessary step in getting a scan. Children who do require contrast agent are not optimally prepared for the scan if they do not receive information about contrast agents. On the other hand, children who do not require contrast agent may become unnecessarily anxious for the scan if they learn about this needle procedure.

Another example is that some skills apply to multiple procedures/exams, but the importance of these specific skills, and the extent to which children need to master them, can differ. This can be understood by the following cases: Jack needs a 10-minute knee MRI without contrast after he fell from his skateboard. Mia needs a 25-minute head MRI with contrast because of a suspected brain tumor. Both Jack and Mia need to learn about lying still. However, Jack will only have to lie still for a short period of time. If Jack does move during the scan, it is possible to do a rescan. Mia has to lie still for a much longer time than Jack, and if she moves after contrast agent injection, a rescan is not possible, but she will likely have to come back to the hospital on another day to redo the entire MRI, which could delay her diagnosis. As such, both Jack and Mia need to be able to lie still for their scan, but the importance of doing so is higher for Mia, and she needs to be able to lay still longer.

Learning goals are one way to describe the things that patients need to learn to be prepared for or become familiarized with in order to have a successful scan. Examples of learning goals are "being familiarized with the sounds of the scanner, "understanding the importance of lying still", and "understanding that metal is not allowed into the MRI room.". A preparation app comprises different games, videos, information modules, AR modules, and other modules that can help patients achieve the required learning goals. In an exemplary AR module, children have to find stickers on an MRI scanner in AR. When they find a sticker, it flips, and educational content related to a specific learning goal is revealed. For example, an image of a head coil is shown, and children are told "During the scan, you will wear a coil that looks a bit like a helmet" (learning goal: "understanding accessories").

Different aspects of tailoring can help to create a training program that optimally prepares patients, such as:

i) Which learning goals are relevant, and how important each learning goal is. This describes what a patient needs to know/master for their particular scan. For example, "understanding that metal cannot go into the MRI room" is a relevant learning goal for a child who needs an MRI scan, but not for a child who needs a CT scan. In the example given above about Jack and Mia, some learning goals will differ between Mia and Jack (e.g., learning about contrast agent will be a learning goal for Mia, but not for Jack), while others are the same (e.g., Jack and Mia both need to learn about laying still).

ii) The threshold required to meet a learning goal. This describes how well a child needs to master a learning goal for their particular scan. For example, laying still is important for almost every scan type, but how well a child needs to be able to lie still depends on scan length; whether a re-scan is possible (e.g., a regular MRI sequence) or not (e.g., for an MRI-sequence after contrast agent injection); whether a re-scan comes with additional health risks (e.g., radiation, as in CT) or not, etc. In the example about Jack and Mia, both need to learn about laying still, but the threshold is higher for Mia than it is for Jack.

iii) The exact content (visuals, sounds, etc.) a patient is exposed to. The exact content of what the child sees/is exposed to in the training program can also be tailored to a specific scan. For example, the goals "being familiarized with MRI accessories" and "being familiarized with the sounds of the scanner" is an important learning goal for both Jack and Mia. However, an MRI exam card comprises different sequences, and these sequences are associated with very different sounds, such that Jack will hear different sounds than Mia. For Mia, the training program may contain the exact sounds associated with her knee exam, while for Jack it may contain the exact sounds associated with his head exam. Likewise, Mia and Jack both need to learn about MRI accessories such as coils, but Jack should see a head coil in the training program, while Mia should see a knee coil.

An example of a list of learning goals with associated thresholds and content is given in Table 1.

Table 1: Example of learning goals with associated thresholds and content for two example patients (Jack and Mia).

| Learning goal | Threshold for Jack | Threshold for Mia | Exact content for Jack | Exact content for Mia |
|---|---|---|---|---|
| Understanding the importance of lying still | medium | high | Scan game with lying still module featuring knee MRI. | Scan game with lying still module featuring knee MRI. |

(continued)

| Learning goal | Threshold for Jack | Threshold for Mia | Exact content for Jack | Exact content for Mia |
|---|---|---|---|---|
| Being familiarized with MRI accessories | medium | medium | Accessory selection in can game: select knee coil, headphones, earplugs. | Accessory selection in can game: select head coil, headphones, earplugs. |
| Being familiarized with the sounds of the scanner | medium | high | Survey, T2_Spair_TSE, COR_2D_TSE (etc.) | Survey, Sag. 3D T1W_TFE, DWIb0_500_100 (etc.) |
| Training breath holds | N/A (scan does not require a breath hold) | N/A (scan does not require a breath hold) | N/A | N/A |
| Understanding that metal is not allowed into the MRI room | medium | medium | Scan game with dressing subgame-identical for all MRI procedures. | |
| etc. | | | | |

The first goal of initial personalization of the app happens before the child has played with the app. It predefines the app module content (for example: MRI or CT workflow and appropriate learning elements of explanation of metals or not (MRI), modality and exam card specific sequence and sounds, breath-hold and contrast elements yes/no; CT or MRI scanner shown in games, etc.). Put differently, the information about the planned procedure determines what learning goals are relevant for the patient. It can also be used to determine the relative importance of those learning goals, as well as the threshold required to meet those learning goals. Moreover, it can be used to dictate the exact nature of the content that is shown to a child to help that child meet the intended learning goals.

An example of how this can be done may be as follows. Initially, high-level information about the patient (e.g., Mia, a 7-year-old patient, requires a brain MRI due to a suspected brain tumour) and required scan type (e.g., taken from the electronic medical record (EMR)) is combined with information about typical exam cards to infer exam characteristics (e.g., Mia required a scan of her head, for 25 minutes, with contrast agent).

Next, for each learning goal for which content (videos, games, etc.) are available, it is determined if that learning goal is relevant, what the relative importance of each learning goal is, and what the envisioned threshold for each learning goal is. This can be determined automatically, based on predefined rules or algorithms (e.g., if scan length is <10 minutes and no contrast is used, threshold for lying still is set to low). Table 2 shows an example of inferred learning goals, thresholds and importance.

Table 2: Inferred learning goals, thresholds and importance

| Learning Goal | Relevant? y/n | Importance low/medium/high | Threshold low/medium/high | Comments |
|---|---|---|---|---|
| Familiarization with sounds | y | medium | medium | N/A |
| Lying still | y | high | high | Long scan with contrast |
| Breath hold in inhale | n | N/A | N/A | N/A |
| etc. | | | | |

Subsequently, the exact content of the app is filled by selecting app modules and specific app content. For example, the relevant sounds can be selected from a database of all sounds. Table 3 shows selected app modules and table 4 shows selected app content.

| App component | Learning goal | Select y/n? | Difficulty level | |
|---|---|---|---|---|
| Table | Sound memory game | Familiarization with sounds | y | high |
| | Lying still game | Lying still | y | difficult |
| | Breath Hold game | Breath holding | n | N/A |
| | etc. | | | |

Selected app modules

Table 4: Selected app content

| Scan sound | Select? y/n |
|---|---|
| CT table in | n |
| MRI survey | y |
| MRI flair | y |
| MRI DWI | y |
| MRI T1W | n |
| etc. | |

Another embodiment of the training program is configured as a game, in which patients need to scan a cartoon elephant. Based on specific scan parameters, the specific content and threshold of the game are set. In this example, the learning goal "understanding accessories (coils)" has been selected as a relevant learning goal for this procedure. A threshold for this learning goal is set, and the exact content (a head coil) is taken from a database containing multiple coil types (in this example, head, anterior, and knee).

According to a second aspect, personalization based on mastery of learning goals is applied, which is discussed in the following. In a first step, an initial version of the app was created based on procedure-specific information, such as the type of scan and the exam card. Once the child starts interacting with the app, the app content can be personalized during game play. Depending on how well learning goals are achieved (e.g., lying still, breath holding, selection of right accessories) the game difficulty can be adapted, and/or different content can be added or recommended to the child. For example, the app can be adapted on that there is less educational content geared towards learning goals that the child already mastered, and more content geared towards learning goals that the child has not yet mastered. The child can also be incentivized to play (sub-)games until the required threshold is reached. This can, for example, be done by providing a child with in-app rewards (points, stars, coins, etc.).

The threshold that children need to reach for each learning goal differs per child: taking the example of laying still, Mia needs to meet a higher threshold than Jack. Game content and difficulty level are adapted until the patient reaches the required threshold.

There are different ways to determine the threshold as well as game progress. One way in which this can be determined is by calculating if the child has reached a performance plateau. It may be that the set threshold lies outside of the capabilities of the child. It can be detected that each child has an S-shaped learning curve that ends in a performance plateau. The height of that plateau differs per person. To establish where someone is on that curve and their performance plateau at least four data points are preferably used. A general learning process can be described with the following formula: SSasymp(t)=Asym+(Perf0-Asym)∗exp(-t/tau)

where t = a numeric vector that represents time trial number 1, 2, ... to complete the training,

Asym = a numeric parameter representing the upper asymptotic value of the model, Perf 0 = a numeric parameter representing the performance at t=0 (i.e., before training), and tau = a numeric parameter representing the learning constant. The relative error between succeeding training trials can be defined by the following formula:

$$rel.error[i]=2*Perf[i]-Perf[i-1]Perf[i]+Perf[i-1]$$

If the relative error is smaller than e.g. 5% or 10%, this may be considered as someone has reached their performance plateau.

When progress is slow or stagnant, additional measures can be taken beyond the game. Two example scenarios are presented:

a) If a child's results are below the required threshold and is stagnant, the games stay on the first easy level until the child's results increase. Once the highest level of the games is achieved, the game stays at that high level of difficulty and prognoses for awake scanning are excellent.

b) If after a predefined amount of time game results are stagnant and below threshold, the child's progress is communicated e.g. to the parents or other relatives or friends, with suggestions on how to help the child achieve learning goals through play with app or with real life games with the parents/relatives/friends. If after time results do not improve, the child's progress as well as the (flat) learning curve is communicated to the radiology staff or caregiver in order to plan for appropriate scan protocols. For example, scheduling a scan with GA, or scheduling an appointment with a specialist for additional face-to-face training.

The learning curve, derived from play data and adapted game difficulty on several parameters, (a learning loop) creates an adaptive and individually personalized learning experience to optimize for learning goals achievement.

There are multiple embodiments in which an awake scan readiness (ASR) score may be used.

In an embodiment the preparedness level of the patient is calculated based on their achievement in the app, compared to the threshold required for the learning goals relevant to their specific scan type. This preparedness level is translated into an ASR score. This easy-to-interpret score (e.g. traffic light) can be used to signal to parents and staff whether the patient is good to go for an awake scan or would require further preparation. The ASR can also contain sub-scores per learning goal that can help inform staff/parents about the exact type of additional preparation/training a child may require. In addition, in severe cases of failure to accomplish the learning goals it can be decided to re-schedule the upcoming scan date altogether. It is also possible to prioritize children based on this support need. Child life specialists often have limited time, so they can devote their time to children who need the extra attention the most. In addition, the screening tool can be used to schedule children for specific locations/time slots (e.g., if a child is likely to require training tools, he or she should be scheduled for a location in which this tool is available). The threshold for learning goals may be fixed and set by the hospital or by the app environment.

Another method for calculating a good threshold is to build a population database with scan type, learning goals, mastery of those learning goals (is this a thing?), and image quality. From that database it can be derived what level mastery is needed for a sufficient image quality. The learning goal mastery could be compared to this population score.

Based on meeting the threshold several decisions can be made:

a) Support Y/N: Does the child needs extra support/training? If they fail to reach the learning goal, they will be flagged as needing extra support. If all goals have been achieved, no additional support is required. The child may also be given an appointment with the child life specialist.

b) Type of support: Based on which goals have not been achieved, it is possible to choose what kind of support is needed. It may be that a child life specialist with a certain expertise is required.

c) The level of support: based on the extent to which a goal has been achieved with respect to the threshold, it is possible to see how much support is needed.

In a further embodiment the break-down of the patient preparedness score can be used to suggest alternative outside-the-app activities that can be undertaken to further prepare the patient. For instance, for parents, fun activities to undertake with their child to prepare them for various aspects, or for staff specific suggestions for on-site training just before the scan (like dedicated time with a miniature scanner (such as the Kitten Scanner) or training by a child life specialist or equivalent focussing on the highlighted learning goals).

In another embodiment the preparedness level of the patient is communicated in a very visual indirect way to all staff interacting with the child to enable an easy means to approach the patient in the optimal way. In particular, using e.g. a badge system or a personalized buddy character, it can be seen in a blink of an eye if the child is e.g. completely ready and good to go, or should get instructed on one particular thing or should be carefully guided throughout. Different elements can signal different preparedness levels, like knowledge versus ability versus emotional readiness.

In another embodiment, the app interacts with the patient to find root causes for stagnant progress and acts on those. For example, if the child is failing the lying still module or showing little evidence of progress, the app could engage the child in a playful Q&A session, in order to ascertain if there is a lack of understanding or skill achievement, or whether there is a more benign reason for stagnation. For instance, the child is simply not lying down to perform the game properly, or the child is entertained with the feedback that is generated by the app when it detects motion.

In a further embodiment, the interaction can be extended to the parent/caregiver to ascertain the child's compliance to correct game play and if indicated the adult's aid can be solicited to the pursuit of correct game play and hence learning goal progress.

In another embodiment, a separate module is added to the game that has the goal of ascertaining whether a child has met learning goals. This can, for example, be a quiz consisting of questions about the learning goals. The outcome of this quiz is subsequently used as input to tailor the game and/or compute the ASR. This quiz may be added to the app as a game module and may contain only questions that match the learning goals that have been pre-selected as being relevant for the scan. Results from the quiz can be used to adapt the ASR and/or to change the difficulty level of games and/or to provide the child with additional content to help the child meet the required learning goals. Alternatively, the child can be explicitly asked whether instructions or game environments were clear.

An example of a separate module to test how well children master learning goals may be as follows. A question asks children "Choose the thing that is not allowed to go into an MRI scanner" to test their mastery of the learning goal "Metal is not allowed in the MRI-scanner". If children answer questions about this learning goal incorrectly, the app can be extended with additional game modules that are associated with this learning goal. In addition, the difficulty level for these learning goals can be lowered, until children are at a point at which the master the learning goal.

There are multiple embodiments that provide a further tailoring and/or personalization of the training program.

In an embodiment, demographic information and/or user preferences are used to further personalize the app. This information can be taken from medical records, manual input, etc. An example is patient age and gender. This information can either be used as a parameter to adapt game content, or calculate ASR or as a parameter to directly choose specific content to personalize the app. For example, if the child scheduled for a scan is a 5-year-old girl, the videos, games and other types of content shown to the child can display a girl with a similar age. This can help children identify with the child, cartoon character or avatar that is shown to have a scan in the app.

In a similar vein, demographic information and/or a child's personal preferences can be taken into account to personalize the app. For example, if a child has chosen 'pink' for ambient experience in previous scans, this information can be used to pre-set the colors of the gameplay (colored lighting in the digital MR room; color of the avatar's clothing, etc.) to pink.

In another embodiment, the app is tailored to hospital-specific parameters or procedures. For example, some hospitals allow children to wear their own clothes, while others require that children wear a hospital gown. This type of hospitals-specific information can be taken into account to further tailor the app. For example, the exact scanner type or accessory type can be shown to the patient, but by showing the exact situation they will encounter when going for their exam. Instead of a generic waiting room they see the waiting room of their hospital, pictures of the actual hospital staff, the exact layout of the MRI room, etc.

In another embodiment, not only the content of the various app modules is tailored, but the app modules themselves are selected or ordered to optimally meet the need of the child. For instance, for a first timer the app is constructed in such a way that first the tailored information movie has to be watched. When receiving the app for a second time the movie may still be accessible but not prioritized first. Similarly, based on their knowledge and experience it may be that certain games may or may not be present at all versus just adjusted in difficulty level.

In another embodiment, physiological measurements (e.g., heart rate, respiration rate, galvanic skin response, etc.), anxiety measures and other measurements are taken into account when determining if a child has met the threshold for a learning goal and/or in calculating ASR.

In another embodiment the time of the day that the exam takes place is taken into account. For example, how a child responds to an exam depends on the circadian rhythm of the child. It is relevant whether the exam takes place early morning or in the afternoon, as a child may be an "early bird" or a "night owl". For an "early bird" morning person it may be an advantage to conduct the exam in the morning. In addition, the events that have happened before the exam (like, lunch, going to school, playing outside) influence the physiological state and cognitive state.

In another embodiment, the intensity or style of the content presentation is personalized. There are many ways to convey the same information or to provide the required (minimum) education. For example, in one case the information can be provided in a direct manner, while in another case it can be presented and taught indirectly. Direct education can be in form of task specific instructions, explicitly listed or made part of the exercise in the app. Indirect education can be via a storytelling approach, where the child is interacting with the app as a part of an entertaining story, and the scan related teachings are peppered as different subtle story elements (e.g. the character needs to lie still not to wake up the baby).

Yet another dimension related to the intensity (level of detail) of the information. In one case, the education can include detailed explanations and exercises, while in another case, the information can be in higher level covering the main elements, and not boring the child with the details.

A third dimension relates to the semantics and choice of the words, sounds, environment used to carry and present the information. In one case, the content can be somewhat emotionally neutral by focusing more on factual information without unnecessary descriptors or adjectives, while in another case the emotionally charged elements (words, sounds, colour, images) can be used.

Having the three dimensions (directness, intensity, affect) at hand, the appropriate content, difficulties, and thresholds can be determined. In one implementation, the available child information can be first used to determine the value for each of the three dimensions, and then based on these the content, difficulty, thresholds, and timing of the app content can be determined. For example, for a child that is good at laying still, the information about importance of not moving can be less detailed, neutral, and more direct. For another child who has difficulty staying still, a more indirect and emotional content can be selected while educating about importance for lying still.

The categorization in terms of the proposed dimensions can be done individually for all elements relevant to the scan. These elements can be, being still, being relaxed, following instructions, and so on. The three-dimension categorization of different elements can be used to quantitatively compare and rank different elements (e.g. by the size of the three-dimensional vector) and consequently to tune the app content accordingly.

In another embodiment, the user's physical characteristics, diagnosis, or health information are taken into account. The scan related information can be used to determine which physical aspects are relevant and to what degree. For example, physical characteristics such as weight, pain in some part of the body, one leg shorter than another, sensitivity to certain frequency of sounds, sensitivity to certain light intensities and frequencies can be all taken into account when determining how the app features can be personalized. In one embodiment, the scan features and the accompanying ambient system characteristics can be used to determine and weight the relationship to the specific user characteristics. Depending on the determined weights, the app content can be adapted. For example, for an overweight child having motion related exercises may have little influence because the source of motion may be the excessive weights (making the patient uncomfortable on the table). For this patient, a better approach may be to train the patient to be distracted or engaged during the scan. By distracting the patient, the motion may be prevented. In this case, the app can focus on making the child accustomed to various games, which can be later used as a distraction during the scan.

Another way to adapt learning thresholds may be as follows. It can be assumed that the thresholds will not be constant from the time the child starts using the app. Maybe, in the beginning, a threshold of one person can be medium, then go to low, then to medium again and so on. In other words, initially the thresholds are defined in terms of their relevance for the exam type, but with time also the app can consider adapting the thresholds to another set of thresholds that are more linked to the current state of the child.

Alternatively, two set of thresholds can be defined. One set is related to the scan type and ambient experience (present in the exam room) boundary conditions, and another set of thresholds is related to the current psychological and physiological state of the child. Both sets of thresholds can then be used while determining the app content. In one simple embodiment, time-based distance from the scheduled scan time can be used which set of thresholds to consider for app adaptation. For example, when the app is first used scan related thresholds can be employed, then personal thresholds can be used, then both thresholds can be equally weighted (used at the same time), and when the scan is days away, the scan thresholds can become dominant again.

In another embodiment, features are adapted according to the required minimum scan quality rather that the best possible behaviour during the scan. Especially for cases, where there are prior scans data, or well-informed expectations, the learning in the app can be accordingly adapted. In a typical case, the app adaptations are done so that maximum possible scan quality is achieved, that is targeting 100% user cooperation. However, if based on some prior information (previous scans) it is know that there is some flexibility in terms of quality of the images (e.g., despite the motion artifacts the disease/condition can be still reliably detected, especially when the scan results are combined and analysed together with the prior data) the app adaptations can be done so that only 50% user collaboration (e.g. lying still) can be achieved. The advantage of such an approach may be the adherence to app usage/learning can be greater (because it will be less challenging and more enjoyable) resulting is some learning (but not complete), which still can be sufficient given the expected minimum scan quality and prior user data.

This concept can be further generalized by having a function that takes as input the user learnings and based on these computes the expected (achievable) scan quality (quality of the images). Further the difference between the required scan quality and the achievable scan quality is computed. The difference signal is then used to determine how features can be adapted. For example, depending on the computed difference, the way the game levels change can be more frequent or less frequent, and the tools/points/awards that become available can be different. The difference can be positive or negative. If the achievable quality is lower than required quality, the user training can continue. if the achievable quality is greater than required quality, the "fun" factors in app can increase.

There are multiple embodiments that deal with managing uncertainties. In an additional embodiment, the training program is adapted based on an estimation of the likelihood that a parent/caregiver can accompany the child into the room of the medical procedure and stay with the child during the procedure. This information can be used as a parameter in calculating the ASR and/or affect the content of information presented to parents/caregivers and children in the app. This parent-in-the-room-likelihood-score can depend on hospital regulations, parent characteristics, including medical characteristics (pregnancy, having an implant) and psychological characteristics (parental anxiety, etc.).

In another embodiment, personalization is adapted based on scenarios in which the exact hospital information is missing or uncertain. For example, in certain cases, there may be doubt about the exact procedure/sequences/scan protocol or there may be multiple options. For example, if a certain scan can be done feet-first or head-first, children should be made aware of multiple scenarios. If data are missing/unavailable or uncertain, the relative importance and threshold for a learning goal can be set accordingly, or the content can contain multiple options, to prepare children for multiple scenarios.

In a further embodiment, there will be automatic and dynamic rescheduling based upon a predefined protocol. In addition, in this embodiment it can be advised that general anesthesia/sedation of the patient is preferred for the upcoming scanning exam.

There are embodiments that provide a further journey personalization, "journey" in this context referring to the complete route the patient "travels" from hearing that a (potentially scary") medical procedure shall be carried out, the patient's preparation, visiting the place of the medical procedure (e.g. a hospital), up to undergoing the actual medical procedure. Although current miniature scanner stories are static (e.g. three stories), an embodiment can be envisioned in which there are an abundant number of stories available to cater to specific learning goals. The ASR score, as well as the details of which learning goals proved to be challenging for patients during app play, may inform the selection of the appropriate stories to display to children. A way for the miniature scanner to identify the child and 'read' ASR and app results could be simply with a code that the app generates that could be scanned at the miniature scanner. Similarly, a child life specialist is informed of which hidden stories to use to support the patient one-on-one upon arrival for the scan. The same code may potentially be scanned at the exam room to select a useful ambient experience theme, as well as personalized guidance of the patient in the scanner during the scan.

In summary, preparing patients through a training program, e.g. a mobile app, can help patients, in particular children, to know what to expect during a medical procedure, such as an imaging scan. The present invention provides a system, device and method to tailor a training program based on specific information about the upcoming medical procedure and to continuously adapt and personalize the training program, for instance its content and difficulty level based on a patient's performance using e.g. individualized thresholds set for specific learning goals.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (2) for training a patient scheduled to undergo a medical procedure, the device comprising:

   an input (21) configured to obtain procedural information comprising information about the scheduled medical procedure;
   a processor (22) configured to construct and execute a personalized training program for training the patient, including

   - constructing an initial training program based on the obtained procedural information;
   - executing the initial training program;
   - obtaining performance information indicating the performance of the patient in the training;
   - updating the training program based on the obtained performance information; and
   - generating control signals for controlling a rendering unit (3) to render visual and/or audible information according to execution of the training program,

   wherein the obtained procedural information is used for constructing the initial training program and/or for updating the training program; and
   an output (23) configured to output the control signals to the rendering unit.

2. Device according to claim 1,
   wherein the processor (22) is configured to construct the initial training program by selecting and ordering one or more training modules and one or more content components based on the obtained procedural information, in particular by selecting and/or adapting and/or ordering one or more of learning goals, difficulty level, prioritization and content of the one or more training modules and/or the one or more content components based on the obtained procedural information.

3. Device according to claim 1 or 2,
wherein the processor (22) is configured to update the training program by adapting one or more of difficulty level, prioritization, content, level of detail, style of presentation, semantics, words, sounds and environment of the one or more training modules and/or the one or more content components based on the obtained performance information, in particular based whether or not and/or to which extent one or more learning goals have been achieved.

4. Device according to any preceding claim,
wherein the input (21) is configured to obtain, as procedural information, information regarding one or more of

- the type of medical procedure,
- the body part of the subject to undergo the medical procedure,
- duration and/or time of the day of the medical procedure,
- details of the medical procedure,
- requirements of the patient during the medical procedure, in particular regarding breath holding, remaining akinetic, and posture,
- travelling time and/or waiting time of the patient before undergoing the medical procedure, and
- the requirement of taking medication and/or administering contrast agent.

5. Device according to any preceding claim,

wherein the input (21) is configured to further obtain patient information comprising information regarding the patient and/or environment information comprising information regarding the environment in which the patient will undergo the scheduled medical procedure, and
wherein the processor (22) is configured to additionally use the obtained patient information and/or the obtained environment information for constructing and/or updating the initial training program.

6. Device according to claim 5,

wherein the input (21) is configured
to obtain, as patient information, information regarding one or more of age, size, weight, gender, health state, medication, medical history, preferences, circadian rhythm, chronotype, pain, sensitivity, problems during a prior medical procedure, frequency and/or intensity of problems during a prior medical procedure, user preferences, prior experience with medical procedures, personality, coping style, mood, frequent emotional states, and/or communication preference, and/or
to obtain, as environment information, information regarding the room in which the patient will undergo the medical procedure including one or more further devices present in the room, colors in the room, persons in the room, layout of the room, clothes worn by the persons and/or the patient during the medical procedure, and other details of the room.

7. Device according to any preceding claim,
wherein the input (21) is configured to obtain the procedural information and/or the patient information and/or the environment information from one or more of a user interface configured to receive user input, an exam card, a medical record, and a database.

8. Device according to any preceding claim,
wherein the processor (22) is configured to obtain, as performance information, information regarding one or more of

- breath holding,
- remaining akinetic,
- being in a predetermined or the same posture,
- required time for solving a task,
- achieved difficulty level,
- coping with emotions including one or more of stress, fear, and anxiety,
- achievements of learning goals,
- physiological measurements including one or more of heart rate, respiration rate, anxiety level, and skin conductance,
- psychological measurements,
- speech,

- sound,
- image and/or video data, and
- number and/or type of errors on a task.

9. Device according to any preceding claim,
wherein the processor (22) is configured to use the performance of the patient in the training to perform one or more of

- determine a patient score indicating the preparedness level of the patient for the scheduled medical procedure;
- determine if the patient needs extra support and which type and level of support;
- scheduling a visit of a doctor or caregiver;
- re-schedule the scheduled medical procedure;
- provide one or more rewards to the patient when one or more learning goals are met;
- suggest one or more further activities of the patient or a caregiver;
- generate and execute interaction, in particular questions and/or suggestions, with the patient and/or a caregiver;
- select content to be presented to the patient;
- collect information to be provided to a caregiver or other person;
- generate a code, identifier or link to information about the performance of the patient and/or regarding parameters that may be used during the scheduled medical procedure.

10. Device according to any preceding claim,
wherein the processor (22) is configured to additionally use, for constructing and/or updating the initial training program, one or more of

- time of the day,
- time up to the scheduled medical procedure,
- required quality of an imaging procedure as scheduled medical procedure,
- likelihood that a caregiver or other person accompanies the patient at the scheduled medical procedure, and
- a predetermined protocol.

11. System (1) for training a patient scheduled to undergo a medical procedure, the system comprising:

- a device (2) for training a patient scheduled to undergo a medical procedure according to any one of the preceding claims; and
- a rendering unit (3) configured to render visual and/or audible information according to control signals received from the device.

12. System according to claim 11, further comprising one or more of:

- a user interface (3) configured to receive user input of the procedural information and/or patient information;
- a procedural information database (5) containing procedural information comprising information about medical procedures including the scheduled medical procedure;
- a patient information database (6) containing patient information comprising information regarding the patient;
- a content components database (7) containing a plurality of content components for constructing a personalized training program, in particular comprising one or more of game components, video components, images, spoken and/or written text components, sound components, movie components for different medical procedures including the scheduled medical procedure, game asset modules, and educational modules;
- a training module database (8) containing a plurality of training modules for constructing a training program; and
- one or more measurement elements (9) configured to measure the patient's performance in the training.

13. System according to claim 11 or 12,

wherein the system (1) is implemented in a computer or a game console or a handheld device, in particular a smartphone, laptop or tablet and/or
wherein the rendering unit (3) comprises a display configured to display visual information to the patient and/or a loudspeaker to output audible information to the patient.

14. Method for training a patient scheduled to undergo a medical procedure, the method comprising:

**EP 4 524 985 A1**

obtaining procedural information comprising information about the scheduled medical procedure;
constructing and executing a personalized training program for training the patient, including

- constructing an initial training program based on the obtained procedural information;
- executing the initial training program;
- obtaining performance information indicating the performance of the patient in the training;
- updating the training program based on the obtained performance information; and
- generating control signals for controlling a rendering unit to render visual and/or audible information according to execution of the training program,

wherein the obtained procedural information is used for constructing the initial training program and/or for updating the training program; and
outputting the control signals to the rendering unit.

15. Computer program comprising program code means for causing a computer to carry out the steps of the method as claimed in claim 14 when said computer program is carried out on the computer.

FIG.1

FIG.2

FIG.3

FIG.4

| | Continuous personalization based on game play data |
|---|---|
| Initial tailoring | Measure performance relative to learning threshold for learning goals based on game play data. → Create updated app (adapt difficulty level, game content, etc). |
| Create initial app Based on procedural data (e.g. exam card containing data on MRI sequences, scan length, contrast need y/n, etc). | |

FIG.5

Europäisches
Patentamt

European
Patent Office

Office européen
des brevets

# EUROPEAN SEARCH REPORT

Application Number

EP 23 19 7862

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | SAINI PRIVENDER ET AL: "Scan Buddy: A Gamified App to Prepare Children for an MRI Scan", 16 June 2022 (2022-06-16), 20220616, PAGE(S) 594 - 612, XP047624564, [retrieved on 2022-06-16] * abstract; claim 1; figures 1-6 * * page 599, paragraph 3 - page 610, paragraph 5 * | 1-15 | INV. G16H20/70 G16H40/20 |

-----

**TECHNICAL FIELDS SEARCHED (IPC)**

G16H

The present search report has been drawn up for all claims

| Place of search | Date of completion of the search | Examiner |
|---|---|---|
| Munich | 4 March 2024 | Menschner, Philipp |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   .......................................................................
& : member of the same patent family, corresponding
   document

EPO FORM 1503 03.82 (P04C01)

# EP 4 524 985 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Scan Buddy: A Gamified App to Prepare Children for an MRI Scan. In Human-Computer Interaction. **SAINI, P.** ; **KOEHN, C.** ; **HEUVELINK, A.** ; **TASAR, O.** ; **VAN VORSTENBOSCH-LYNN, E.** ; **NAUTS, S.** ; **TROUT, A. T.** Theoretical Approaches and Design Methods: Thematic Area, HCI 2022, Held as Part of the 24th HCI International Conference, HCII 2022, Virtual Event. Cham: Springer International Publishing, 26 June 2022, 594-612